# EUROPEAN PATENT SPECIFICATION

(11) **EP 2 108 961 B1**
(45) Date of publication and mention of the grant of the patent: **20.05.2015**
(21) Application number: 09005263.0
(22) Date of filing: 09.04.2009
(51) Int. Cl.: G01N 33/92, G01N 33/52, C12Q 1/60

(54) **Dry analytical element for measurement of high density lipoprotein cholesterol**
Trockenes analytisches Element zur Messung von hochdichtem Lipoproteincholesterin
Elément analytique sec pour la mesure de cholestérol de lipoprotéine à forte densité

(30) Priority: 10.04.2008 JP 2008102247
(43) Date of publication of application: 14.10.2009
(73) Proprietor: FUJIFILM Corporation, Tokyo 106-8620 (JP)
(72) Inventor: Jumawid, Mariejoy, Asaka-shi Saitama 351-8585 (JP); Kawasaki, Kazuya, Asaka-shi Saitama 351-8585 (JP); Inomata, Hiroko, Asaka-shi Saitama 351-8585 (JP)
(74) Representative: Hoffmann Eitle

(56) References cited:
- EP-A- 0 597 268
- EP-A- 0 699 767
- EP-A- 1 114 870
- EP-A- 1 342 792
- EP-A- 1 505 393
- EP-A- 1 854 894
- EP-A2- 1 028 319
- WO-A-2006/067424
- WO-A-2006/118199
- WO-A1-2006/023678
- US-A- 5 786 164
- US-A1- 2005 074 828
- US-A1- 2006 063 267

## Description

### Technical Field

The present invention relates to a dry analytical element for the measurement of high density lipoprotein cholesterol (HDL-C) in a specimen. The level of high density lipoprotein cholesterol in serum is known to serve as an index useful for predicting the onset of arteriosclerotic diseases.

### Background Art

Lipids present in blood are incorporated in the structure of lipoprotein, except for free fatty acid bound with albumin, and are present as chylomicron (CHM), very low density lipoprotein (VLDL), low density lipoprotein (LDL), high density lipoprotein (HDL), and the like. Cholesterol therein is particularly distributed in VLDL, LDL, and HDL. HDL is allegedly a preventive factor for heart diseases attributed to arteriosclerosis. Thus, the measurement of high density lipoprotein cholesterol (HDL-C), instead of HDL, clinically has a more important meaning. Ultracentrifugation, electrophoresis, and precipitation methods are widely known as current methods for measuring HDL-cholesterol.

The ultracentrifugation method is not suitable for daily examination for such reasons that it requires a long time for separation procedures; and inexpensive measurement cannot be expected because an apparatus itself is expensive. The electrophoresis method still has a problem in light of quantification for such reasons that: its separation ability differs depending on a difference in electrophoresis support medium; and the method differs depending on use conditions and detection reagents used. Thus, the precipitation method is widely used as current daily examination.

The precipitation method is a method comprising using, for example, a combination of a polyanion and a divalent metal ion, as a precipitating reagent to precipitate CHM, LDL, and VLDL and measuring cholesterol in HDL, that is, HDL-cholesterol, remaining in the supernatant by use of a chemical reagent or enzyme. A combination of a sulfate polysaccharide and an alkaline earth metal ion or a divalent metal ion other than alkaline earth, an inorganic polyanion salt, polyethylene glycol, and so on, which have been well known since the early 1960s by T. Nakai, "HDL-Metabolism, Assay Methods and Clinical Application" (Chugaiigaku Co., Ltd., 1986) and other various documents and textbooks, are widely used as the precipitating reagent Specific examples of the precipitating reagent include a heparin-calcium reagent, a dextran sulfate-magnesium reagent, and a phosphotungstic acid-magnesium reagent

The precipitation method is a method in which serum is mixed with the precipitating reagent and left for certain period of time, and after centrifugation at approximately 3000 revolutions per minute, an aliquot of the supernatant portion is separated and subjected to chemical reaction or enzyme reaction to quantify HDL-cholesterol.

The precipitation method presents problems derived from the precipitating reagent and problems derived from centrifugation procedures. Therefore, JP Patent Publication (Kokai) Nos. 55-78254A (1980), 55-93065A (1980), 61-263467A (1986), and 62-19768A (1987), JP Patent Publication (Kokoku) No. 1-39553B (1989), etc. have described various methods for improving precipitating agents for enhancing precipitation efficiency.

The major disadvantage of the conventional precipitation method is that the use of a reagent rich in triglyceride sometimes results in the partial floating of precipitates after centrifugation. Therefore, the precipitation method presents such a big problem that the adjustment of centrifugation conditions is required. In a method using phosphotungstate-magnesium ions, precipitation sometimes varies depending on the pH of the solution. Therefore, the method presents such a problem that the strict adjustment of pH is required.

When a centrifuged supernatant, particularly a small amount of the supernatant, is separated, the boundary region between precipitates and the supernatant is difficult to determine by visual observation. Therefore, quantitative analysis precision is sometimes lowered due to reproducibility and precision problems and variations among individuals. It has been demanded to improve these disadvantages attributed to centrifugation procedures.

A direct method that does not require these complicated procedures and is available for an automatic analyzer has spread rapidly in recent years. For example, JP Patent Publication (Kokai) No. 8-131197A (1996) has disclosed a method comprising sufficiently reacting sulfated cyclodextrin used as an aggregating agent with lipoproteins other than HDL, then allowing an enzyme labeled with polyoxyethylene glycol to act thereon, and measuring cholesterol in HDL. WO98/26090 has disclosed a method comprising a first step of eliminating lipoproteins other than HDL by use of catalase and a second step of measuring HDL-C by use of an activator that specifically acts on HDL. JP Patent Publication (Kokai) No. 9-96637A (1997) has described a method comprising initially allowing an antibody reacting with lipoproteins other than HDL to act thereon, subsequently dissolving HDL, and measuring cholesterol in HDL. Furthermore, WO2004/035816 has disclosed that HDL-C has been accurately measured by allowing HDL-C to react with enzyme that preferentially acts on it or by reacting HDL-C in an aqueous medium that contains a nonionic surfactant, polyanion and albumin. Dextran sulfate was used as such polyanion. Dextran sulfates having molecular weights of 40,000, 80,000, 200,000, 500,000, 1,000,000, and 2,000,000 were used. This HDL-C measurement method enables a favorable measurement of HDL-C contained in liquid. However, if this method is applied to a dry analytical element, it does not have selectivity for HDL-C, and thus, it is not able to measure the HDL-C.

In the field of dry chemistry, a novel test piece for a dry process using the direct method has been developed, and it is described in JP Patent No. 3686326. JP Patent Publication (Kokai) No. 2005-137360A has disclosed that the use of lipase derived from *Candida rugosa* improves such selectivity. However, any of the test pieces cannot completely remove cholesterol in lipoproteins other than HDL.

EP 1 854 894 A1 relates to a reagent kit for HDL-C detection and a dry analytical element for HDL-C detection as well as a method for measuring HDL-C in a specimen. In the method for measuring high density lipoprotein cholesterol (HDL-C) in a body fluid test sample, cholesterol esterase derived from Schizophyllum commune or Pseudomonas sp. and cholesterol oxidase derived from Pseudomonas sp. are used to generate hydrogen peroxide from HDL-C, and thereby HDL-C is selectively measured.

WO 2006/118199A relates to a method for determination of HDL cholesterol, a reagent for the determination and a kit for the determination. The method for determination of cholesterol in a high-density lipoprotein in a sample comprises the steps of reacting the sample with i) a cholesterol ester hydrolase and a cholesterol oxidase or ii) a cholesterol ester hydrolase, an oxidized coenzyme and a cholesterol dehydrogenase in an aqueous medium containing a specific nitrogenated surfactant having a structure of an amine or ammonium salt and a polyanion to produce hydrogen peroxide or a reduced coenzyme, and determining hydrogen peroxide or the reduced coenzyme.

US 2006/0063267 A1 and WO 2006/023678 A1, which belong to the same patent family, relate to a bodily fluid analyzer including a dry test strip impregnated with a reagent providing a non-precipitating reaction to exclude non-desired analytes. The reagent complexes the non-desired analytes so they remain in solution, but cannot participate in the test reaction. Red blood cells are removed from the detection area by slowing their vertical movement and stopping flow when the detection membrane is saturated.

EP 1 028 319 A2 relates to an assay device for measuring the concentration of a soluble analyte, such as HDL-associated cholesterol, in a fluid sample containing interfering compounds, such as LDL- or VLDL-associated cholesterol, which can be selectively precipitated. The device includes a sieving matrix capable of separating soluble and precipitated material migrating through the matrix, and a reservoir which holds a precipitating agent which is effective, within a given concentration range, to selectively precipitate the interfering compounds. The reservoir is designed to delay the release of agent, on contact with the fluid sample, to maintain the concentration of precipitating agent in contact with the fluid sample within the given concentration range. The device additionally includes an assay pad in which the soluble analyte present in the fluid sample can be assayed.

US 5 786 164 A relates to a method useful in separating non-high density lipoproteins, referred to as "non-HDLs", from biological fluids containing them. A porous carrier is provided which contains a non-HDL precipitating agent.

### Disclosure of the Invention

It is an object of the present invention to provide a dry analytical element for the measurement of HDL-C, in which the interference of lipoproteins other than HDL is suppressed.

As a result of intensive studies directed towards achieving the aforementioned object, the present inventer has found that lipoproteins other than a high density lipoprotein (HDL) can be stably aggregated and cholesterol contained in HDL can be efficiently and selectively measured by using dextran sulfate with a molecular weight of 36,000 to 50,000, when such lipoproteins other than HDL are allowed to aggregate in the presence of an nonionic surfactant of 3 to 22 g/m² and a buffer of 0.1 to 40 mmol/m². The present invention has been completed based on such findings.

That is to say, the present invention provide a dry analytical element for the measurement of a high density lipoprotein cholesterol, which has at least one layer comprising an nonionic surfactant of 3 to 22 g/m², a buffer of 0.1 to 40 mmol/m², dextran sulfate with a molecular weight of 36,000 to 50,000, a divalent metal ion and reagents for measuring high density lipoprotein cholesterol, on a support.

Preferably, the nonionic surfactant is a single type of nonionic surfactant, or a mixture of two or more types of nonionic surfactants.

Preferably, the nonionic surfactant is a polyoxyethylene derivative and/or a polyoxyethylene-polyoxypropylene condensate.

Preferably, one of the nonionic surfactants is a nonionic surfactant that selectively solubilizes a high density lipoprotein.

Preferably, one of such nonionic surfactants is a nonionic surfactant that inhibits dissolution of lipoproteins other than the high density lipoprotein.

Preferably, the buffer is present in at least one layer.

Preferably, the nonionic surfactant is present in at least one layer.

Preferably, the dextran sulfate is present in at least one layer.

Preferably, the dry analytical element for the measurement of high density lipoprotein cholesterol of the present invention comprises at least cholesterol esterase and cholesterol oxidase as reagents for measuring the high density lipoprotein cholesterol.

Preferably, the dry analytical element for the measurement of high density lipoprotein cholesterol of the present invention further comprises peroxidase and chromogens as reagents for measuring the high density lipoprotein cholesterol. Preferably, as such chromogens, 4-aminoantipyrine or a derivative thereof, and a Trinder's reagent coupling with the 4-aminoantipyrine or the derivative thereof are used.

Preferably, a Good's buffer is used as the aforementioned buffer. The Good's buffer is
characterized in that it has various types of aminoethanesulfonic acids and aminopropanesulfonic acid derivatives having a Zwitter ion structure, so that it can function as a biochemical buffer. The Good's buffer has a pH value ranging from pH 5.5 to pH 11.0. Representative Good's buffers include MES, Bis-Tris, ADA, PIPES, ACES, MOPSO, BES, MOPS, TES, HEPEES, DIPSO, TAPSO, POPSO, HEPPSO, EPPS, Tricine, Bicine, TAPS, CHES, CAPSO, and CAPS. More preferred Good's buffers that can be used herein include 2-Morpholinoethanesulfonic acid, monohydrate (MES) and *N-*Tris(hydroxymethyl)methyl-2-aminoethanesulfonic acid (TES).

In the present invention, as aggregating agents that aggregate lipoproteins other than HDL, dextran sulfate with a molecular weight of 36,000 to 50,000 and a divalent metal can be used. Preferably, the divalent metal is magnesium.

Preferably, the dry analytical element for the measurement of high density lipoprotein cholesterol of the present invention has at least one adhesive layer and one porous spreading layer on a water-impermeable support.

According to the dry analytical element for the measurement of HDL-C of the present invention, it became possible to suppress the interference of lipoproteins other than HDL in the measurement of HDL-C.

### Brief Description of the Drawings

Figure 1 shows the results of evaluation of VLDL interference using the dry analytical element of the present invention comprising dextran sulfate having a molecular weight of 36,000 to 50,000, and the results of evaluation of VLDL interference using dry analytical elements comprising dextran sulfates each having a molecular weight of 500,000 or 5,000.
Figure 2 shows the results of evaluation ofLDL interference using the dry analytical element of the present invention comprising dextran sulfate having a molecular weight of 36,000 to 50,000, and the results of evaluation of LDL interference using dry analytical elements comprising dextran sulfates each having a molecular weight of 500,000 or 5,000.
Figure 3 shows the influence of the concentration of a buffer contained in a dry analytical element upon VLDL interference.
Figure 4 shows the influence of the concentration of a buffer contained in a dry analytical element upon LDL interference.
Figure 5 shows the influence of the amount of a nonionic surfactant contained in a dry analytical element upon VLDL interference.
Figure 6 shows the influence of the amount of a nonionic surfactant contained in a dry analytical element upon LDL interference.

### Best Mode for Carrying Out the Invention.

The embodiments of the present invention will be described below.

The dry analytical element for the measurement of HDL-C of the present invention is
**characterized in that** dextran sulfate with a molecular weight of 36,000 and 50,000 is used in the presence of a nonionic surfactant of 3 to 22 g/m², a buffer of 0.1 to 40 mmol/m² and a divalent metal ion, so as to allow lipoproteins other than HDL to stably aggregate, thereby selectively measuring HDL-C.

HDL-C contained in a body fluid sample can be measured using the dry analytical element for the measurement of HDL-C of the present invention. As a body fluid sample, blood, urine, and the like can be used. As a body fluid sample, blood or urine may be directly used, or a body fluid that has been subjected to an appropriate pre-treatment may also be used.

Next, reagents used in the dry analytical element of the present invention will be explained.

In the present invention, as an aggregating agent that aggregates lipoproteins other than HDL, dextran sulfate with a molecular weight between 36,000 and 50,000 is used. As such aggregating agents that aggregate lipoproteins other than HDL, a combination of a sulfated polysaccharide and an alkaline earth metal ion or a divalent metal ion other than alkaline earth, an inorganic polyanion salt, polyethylene glycol, and so on, have been known since the early 1960s by T. Nakai, "HDL-Metabolism, Assay Methods and Clinical Application" (Chugaiigaku Co., Ltd., 1986), and various other publications and textbooks. Of these precipitating reagents, examples of a combination of a sulfated polysaccharide and a metal ion include precipitating reagents such as a dextran sulfate-calcium (2+) ion complex described in "Journal of Laboratory and Clinical Medicine" Vol. 82, p. 473- (1973), and a dextran sulfate-magnesium (2+) ion complex described in "J. Lipid Res." Vol. 11, p. 583-595 (1970), "Clin. Chem." Vol. 24, p. 931-933 (1978). In the present invention, a combination of dextran sulfate with a molecular weight between 36,000 and 50,000 and a magnesium ion is particularly preferably used. An example of such dextran sulfate with a molecular weight between 36,000 and 50,000 is MP Biomedicals (manufacturer's code: 160110).

In the present invention, a buffer is used at an amount of 0.1 to 40 mmol/m². Example of such a buffer include Good's buffers described in "Biochemistry" Vol. 5 (No. 2), p. 467-477 (1966). These pH buffers can be selected with reference to the descriptions of documents such as "Basic Experimental Methods of Proteins and Enzymes" (T. Horio et al., Nankodo Co., Ltd.,1981) and "Biochemistry" Vol. 5. More preferably, 2-Morpholinoethanesulfonic acid, monohydrate (MES) or *N*-Tris(hydroxymethyl)methyl-2-aminoethanesulfonic acid (TES) is used.

The pH of the aforementioned buffers is determined depending on the optimum pH of the enzyme used, and it is adjusted to preferably pH 5.0 to 8.0, and more preferably pH 6.0 to 7.0. The concentration of such a buffer is preferably adjusted to be 0.1 to 40 mmol/m² for stable aggregation of lipoproteins other than HDL.

In the present invention, a nonionic surfactant is used. In the present invention, a surfactant that preferentially dissolves HDL is preferably used. As such a surfactant that preferentially dissolves HDL, a polyoxyethylene derivative can be used. Examples of commercially available products of such nonionic surfactants include Noigen EA-157, Emulgen B66 (manufactured by Kao Corp.), and Emulgen A90 (manufactured by Kao Corp.).

In the present invention, a nonionic surfactant that inhibits the dissolution of lipoproteins other than HDL can be used. An example of such a surfactant that inhibits the dissolution of lipoproteins other than HDL is a polyoxyethylene-polyoxypropylene condensate. Examples of commercially available products of such polyoxyethylene-polyoxypropylene condensates include Pluronic series (manufactured by ADEKA Corp.).

The amount of a surfactant that selectively dissolves HDL and/or a surfactant that inhibits the dissolution of lipoproteins other than HDL is 3.0 to 22 g/m² in a dry analytical element

Enzymes used in the dry analytical element of the present invention are cholesterol esterase and cholesterol oxidase. Cholesterol esterase derived from *Schizophyllum commune* or Pseudomonas sp. is used as the cholesterol esterase of the present invention. Cholesterol esterase derived form *Schizophyllum commune* is particularly preferable. Cholesterol oxidase derived from Pseudomonas sp. is used as the cholesterol oxidase of the present invention. Any of the enzymes used in the present invention may be an enzyme derived from each of the microorganisms or may be a recombinant product produced by a well known method.

An example of the cholesterol esterase derived from *Schizophyllum commune* is COE-302 manufactured by TOYOBO Co., Ltd. Examples of the cholesterol esterase derived from Pseudomonas sp. include COE-311, LPL-312, and LPL-314 manufactured by TOYOBO Co., Ltd. and CEN manufactured by Asahi Kasei Corp. Examples of the cholesterol oxidase derived from Pseudomonas sp. include CHO-PEL and CHO-PEWL manufactured by Kikkoman Corp.

In addition to the aforementioned reagents, a well known enzyme reagent, chromogens, and a pH buffer can be used as reagents for cholesterol detection in the present invention.

Specifically, an example of the enzyme is peroxidase. Examples of the chromogens include 4-aminoantipyrine (4-AA) and a phenolic or anilinic Trinder's reagent that develops color though hydrogen-donating coupling therewith. Preferably, the Trinder's reagent is an anilinic reagent Examples thereof include ADPS, ALPS, TOPS, ADOS, DAOS, HDAOS, MAOS, and TOOS manufactured by DOJINDO Laboratories.

The dry analytical element of the present invention may preferably comprise at least one adhesive layer and at least one porous spreading layer on a water-impermeable support.

The aforementioned porous layer may be fibrous or non-fibrous. The porous layer functions as a spreading layer for a liquid sample, and therefore, it is preferably a layer having a liquid measurement action. The liquid measurement action is an action by which the liquid sample supplied by spotting onto the surface of the layer is spread in an almost constant amount per unit area in the direction of the surface of the layer without substantially unevenly distributing components contained in the sample. The spreading layer can comprise a hydrophilic polymer or a surfactant as described in JP Patent Publication (Kokai) Nos. 60-222770A (1985), 63-219397A (1988), and 62-182652A (1987) in order to adjust a spreading area, a spreading rate, and so on.

Layers made of polyester fibers as those typified by JP Patent Publication (Kokai) Nos. 55-164356A (1980), 57-66359A (1982), and 60-222769A (1985), etc. are preferable as the fibrous porous layer. Layers made of organic polymers such as polysulfonic acid are preferable as the non-fibrous porous layer.

The adhesive layer has a function of adhering the water-impermeable support and the porous layer. Hydrophilic polymers such as gelatin and derivatives thereof (e.g., phthalated gelatin), cellulose derivatives (e.g., hydroxypropylcellulose), agarose, acrylamide polymers, methacrylamide polymers, and copolymers of acrylamide or methacrylamide and a variety of vinyl monomers can be utilized.

An aqueous solution containing the hydrophilic polymer is evenly coated to the support by a well known method. A method known in the art can be used as the coating method. For example, dip coating, extrusion coating, doctor coating, hopper coating, curtain coating, or the like can be selected appropriately and used for the coating.

The porous layer can be coated onto the adhesive layer. Preferably, a fabric or porous film provided in advance as knitting is laminated onto the adhesive layer. A laminating method is a method as described in JP Patent Publication (Kokai) No. 55-164356A (1980), in which the surface of the adhesive layer containing the hydrophilic polymer is made uniformly wet with water, and the fabric or porous film is laid on the surface and allowed to adhere thereto by slight and almost uniform pressure. The thickness of the adhesive layer is preferably 0.5 to 50 µm, and more preferably 1 to 20 µm.

Preferable materials for a light-permeable support are polyethylene terephthalate, polystyrene, and cellulose ethers such as cellulose triacetate. To allow a water absorption layer of the hydrophilic layer, a detection layer, a substantially non-porous reagent layer, and so on to firmly adhere to the support, the support is usually provided with an undercoat layer or subjected to a hydrophilizing treatment. The thickness of the support is not particularly limited and is preferably 10 to 1,000 µm, and more preferably 300 to 800 µm. When the support is light-permeable, final detection may be performed on the support side or on the porous layer side. When the support is light-impermeable, detection is performed from the porous layer side.

Next, a reagent composition for cholesterol measurement and a reagent composition that causes an optical change, which are used in the dry analytical element of the present invention, will be described.

The reagent compositions may be contained in the first porous layer or may be contained both in the adhesive layer and in the porous layer. Alternatively, all or most of the reagent compositions may be contained in either of the layers or may be contained in layers other than the adhesive layer and the porous layer.

In a dry analytical element used in HDL-C detection, the aforementioned nonionic surfactant (preferably, a surfactant that preferentially dissolves HDL, and/or a surfactant that inhibits the dissolution of lipoproteins other than HDL) is supplied in an amount of 3 to 22 g per m².

The pH of the aforementioned buffer is determined depending on the optimum pH of the enzyme used, and it is adjusted to preferably pH 5.0 to 8.0, and more preferably pH 6.0 to 7.0. In the dry analytical element, a dry agent is present in at least one layer. Preferably, the aforementioned buffer is used in an amount of 0.1 to 40 mmol per m².

In the dry analytical element used in HDL-C detection, dextran sulfate (MW = 36,000 to 50,000) combined with a magnesium ion is preferably used as an aggregating agent that aggregates lipoproteins other than HDL. Such an aggregating agent is used in an amount of preferably 0.05 to 10 g per square meter in terms of dextran sulfate and 0.01 to 20 g per square meter in terms of magnesium chloride hexahydrate. It is used in an amount of more preferably 0.1 to 5 g/m² in terms of dextran sulfate and 0.5 to 10 g/m² in terms of magnesium chloride hexahydrate.

In the dry analytical element used in HDL-C detection, any of the enzymes (cholesterol esterase derived from *Schizophyllum commune* or Pseudomonas sp. and cholesterol oxidase derived from Pseudomonas sp.) are used in an amount of preferably 0.1 to 20 kU per square meter, and more preferably 0.5 to 10 kU per square meter.

The origin of the aforementioned peroxidase is not particularly limited. It is preferably derived from horseradish. The amount of the peroxidase used is preferably 1 to 100 kU/m², and more preferably 10 to 80 kU/m².

A combination of 4-aminoantipyrine (4-AA) and the reagent that develops color through coupling therewith is preferable as such chromogens. Particularly preferably, DAOS is used. The amount of each of the chromogens (4-AA and a hydrogen-donating coupling agent) used is preferably 0.1 to 10 g/m², and more preferably 0.3 to 5 g/m².

Other reagent compositions contained in the dry analytical element for HDL-C detection can optionally comprise a stabilizer, a cross-linking agent (a hardener or a curing agent), a polymer, and the like. These reagents can be contained in the adhesive layer or the porous layer of the dry analytical element of the present invention.

For example, the dry analytical element of the present invention can be cut into small pieces such as a square of approximately 5 mm to approximately 30 mm on a side or a circle having almost the same size and can be used as a chemical analysis slide placed in a slide mount described in JP Patent Publication (Kokoku) No. 57-283331B (1982) (corresponding U.S. Patent No. 4,169,751), JP Utility Model Publication (Kokai) No. 56-142454U (1981) (corresponding U.S. Patent No. 4,387,990), JP Patent Publication (Kokai) No. 57-63452A (1982), JP Utility Model Publication (Kokai) No. 58-32350U (1983), JP Patent Publication (Kohyo) No. 58-501144A (1983) (corresponding International Publication No. WO083/00391), etc. This is preferable from the viewpoint of production, packaging, transport, storage, measurement procedures, and so on. Depending on the purpose of usage, the dry analytical element is placed in a long tape form in a cassette or magazine for use. Alternatively, the small piece thereof is placed in a container with an opening for use or attached to or placed in an aperture card for use. Alternatively, the small cut piece can also be used directly.

For example, an aqueous liquid sample solution is spotted in a range from approximately 2 µL to approximately 30 µL, preferably 4 µL to 15 µL, onto the porous spreading layer for a liquid sample in the dry analytical element of the present invention. The dry analytical element on which the sample solution has been spotted is incubated at a constant temperature ranging from approximately 20°C to approximately 45°C, and preferably approximately 30°C to approximately 40°C, for 1 to10 minutes. Color development or discoloration within the dry analytical element is reflex-measured from the light-transmitting support side. A calibration curve prepared in advance can be used to determine the amount of the test substance in the specimen according to the principle of colorimetry.

The measurement procedures can be practiced according to exceedingly easy procedures using a chemical analyzer described in JP Patent Publication (Kokai) Nos. 60-125543A (1985), 60-220862A (1985), 61-294367A (1986), and 58-161867A (1983) (corresponding U.S. Patent No. 4,424,191), etc. to achieve quantitative analysis with high precision. Depending on purposes and necessary precision, the degree of color development may be assessed by visual observation to perform semiquantitative measurement

The dry analytical element of the present invention is stored and preserved in a dry state until analysis. Therefore, the reagents do not have to be prepared before use. Moreover, reagents in a dry state generally have high stability. Therefore, the method of the present invention is more convenient and rapid than a so-called solution method in which reagent solutions must be prepared before use. Moreover, the method of the present invention is also excellent as an examination method capable of rapid examination with high precision using a trace amount of liquid samples.

The present invention will be described further specifically in the following examples.
However, these examples are not intended to limit the present invention.

### Examples

### Example 1: Dry analytical element for measurement ofHDL cholesterol

A gelatin aqueous solution was coated to a 180-µm polyethylene terephthalate colorless transparent smooth film previously coated with gelatin, resulting in a thickness after drying of 14 µm, and the film was then dried. Subsequently, water was supplied in an amount of approximately 30 g/m² to the entire surface of the aforementioned film, so as to wet the film. Thereafter, a 36-gauge tricot knitting fabric made of polyester spun yarn corresponding to 50 deniers was slightly pressed and stacked thereon, followed by drying. Subsequently, an aqueous solution with a composition as described below was coated onto the fabric, followed by drying.

| | |
|---|---|
| MES buffer (pH 6.6) | 10 mmol/m² |
| Cholesterol esterase (derived from *Schizophyllum commune)* | 3.7 kU/m² |
| Cholesterol oxidase (derived from Pseudomonas sp.) | 1.1 kU/m² |
| Peroxidase | 60 kU/m² |
| 4-aminoantipyrine (manufactured by Wako Pure Chemical Industries, Ltd.) | 0.3 g/m² |
| DAOS (manufactured by DOJINDO Laboratories) | 0.5 g/m² |
| Emulgen A90 (manufactured by Kao Corp.) | 6.2 g/m² |
| Pluronic F-88 (manufactured by ADEKA Corp.) | 1.3 g/m² |
| Dextran sulfate (MW: 36,000 to 50,000) (manufactured by Wako Pure Chemical Industries, Ltd.) | 1.4 g/m² |
| Magnesium chloride hexahydrate (manufactured by Wako Pure Chemical Industries, Ltd.) | 8.8 g/m² |

### Example 2: Evaluation ofHDL cholesterol measurement

Using the dry analytical element of Example 1, the measurement of HDL cholesterol was evaluated.
(1) Preparation of calibration curve As a standard solution, distilled water (HDL cholesterol: 0 mg/dL) and serum (HDL cholesterol: 20, 50, and 80 mg/dL) were spotted in an amount of 10 µL to the dry analytical element of Example 1. A calibration curve showing the relationship between an HDL cholesterol concentration and the absorbance at 600 nm was prepared.
(2) Evaluation of VLDL and LDL interference

A purified product of VLDL or LDL was added at a ratio of 1 : 1 to a human serum specimen. Distilled water was then added thereto to result in the final concentration of VLDL of 20 mg/dL and that of LDL of 60 mg/dL, so as to prepare a specimen. Thereafter, 10 µL, of the prepared specimen was added to the dry analytical element, followed by incubation at 37°C for 6 minutes. HDL cholesterol was calculated based on the absorbance obtained 6 minutes later and the prepared calibration curve. The serum specimen to which VLDL or LDL had been added and the serum specimen to which lipoproteins other than HDL had not been added were plotted, and the interference of VLDL or LDL was evaluated.

### Comparative example 1: Dry analytical element for measurement of HDL cholesterol

A gelatin aqueous solution was coated to a 180-µm polyethylene terephthalate colorless transparent smooth film previously coated with gelatin, resulting in a thickness after drying of 14 µm, and the film was then dried. Subsequently, water was supplied in an amount of approximately 30 g/m² to the entire surface of the aforementioned film, so as to wet the film. Thereafter, a 36-gauge tricot knitting fabric made of polyester spun yarn corresponding to 50 deniers was slightly pressed and stacked thereon, followed by drying. Subsequently, an aqueous solution with a composition as described below was coated onto the fabric, followed by drying.

| | |
|---|---|
| MES buffer (pH 6.6) | 10 mmol/m² |
| Cholesterol esterase (derived from *Schizophyllum commune)* | 3.7 kU/m² |
| Cholesterol oxidase (derived from Pseudomonas sp.) | 1.1 kU/m² |
| Peroxidase | 60 kU/m² |
| 4-aminoantipyrine (manufactured by Wako Pure Chemical Industries, Ltd.) | 0.3 g/m² |
| DAOS (manufactured by DOJINDO Laboratories) | 0.5 g/m² |
| Emulgen A90 (manufactured by Kao Corp.) | 6.2 g/m² |
| Pluronic F-88 (manufactured by ADEKA Corp.) | 1.3 g/m² |
| Dextran sulfate (MW: 500,000) (manufactured by Wako Pure Chemical Industries, Ltd.) | 1.4 g/m² |
| Magnesium chloride hexahydrate (manufactures by Wako Pure Chemical Industries, Ltd.) | 8.8 g/m² |

### Comparative example 2: Dry analytical element for measurement ofHDL cholesterol

A gelatin aqueous solution was coated to a 180-µm polyethylene terephthalate colorless transparent smooth film previously coated with gelatin, resulting in a thickness after drying of 14 µm, and the film was then dried. Subsequently, water was supplied in an amount of approximately 30 g/m² to the entire surface of the aforementioned film, so as to wet the film Thereafter, a 36-gauge tricot knitting fabric made of polyester spun yarn corresponding to 50 deniers was slightly pressed and stacked thereon, followed by drying. Subsequently, an aqueous solution with a composition as described below was coated onto the fabric, followed by drying.

| | |
|---|---|
| MES buffer (pH 6.6) | 10 mmol/m² |
| Cholesterol esterase (derived from *Schizophyllum commune)* | 3.7 kU/m² |
| Cholesterol oxidase (derived from Pseudomonas sp.) | 1.1 kU/m² |
| Peroxidase | 60 kU/m² |
| 4-aminoantipyrine (manufactured by Wako Pure Chemical Industries, Ltd.) | 0.3 g/m² |
| DAOS (manufactured by DOJINDO Laboratories) | 0.5 g/m² |
| EmulgenA90 (manufactured by Kao Corp.) | 6.2 g/m² |
| Pluronic F-88 (manufactured by ADEKA Corp.) | 1.3 g/m² |
| Dextran sulfate (MW: 5,000) (manufactured by Wako Pure Chemical Industries, Ltd.) | 1.4 g/m² |
| Magnesium chloride hexahydrate (manufactured by Wako Pure Chemical Industries, Ltd.) | 8.8 g/m² |

### Comparative example 3: Evaluation ofHDL cholesterol measurement

HDL cholesterol was measured by the same method as the measurement method of Example 2, with the exception that the dry analytical element of Comparative example 1 was used instead of that of Example 1.

### Comparative example 4: Evaluation of HDL cholesterol measurement

HDL cholesterol was measured by the same method as the measurement method of Example 2, with the exception that the dry analytical element of Comparative example 2 was used instead of that of Example 1.

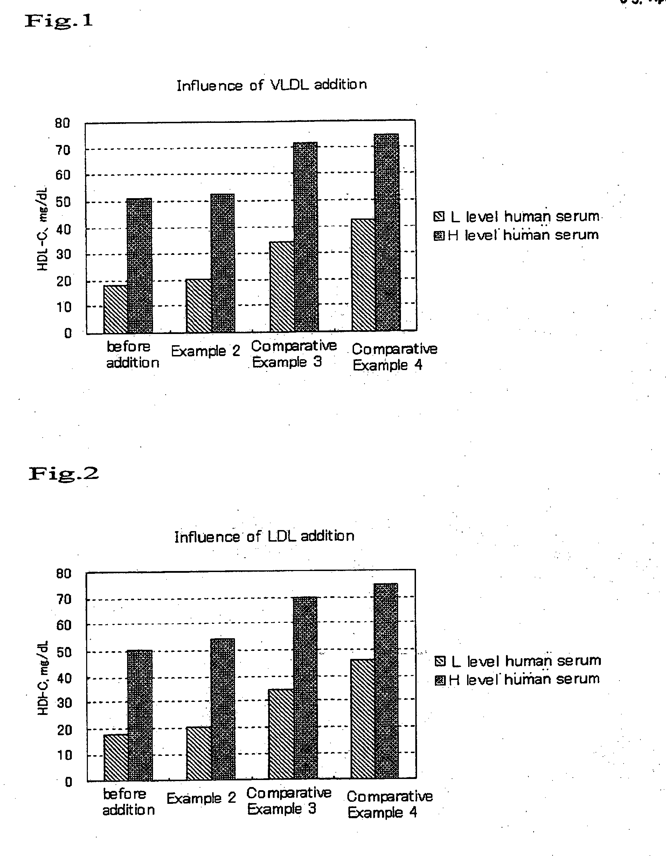

The HDL-C measurement and VLDL/LDL interference of each of Example 2 and Comparative examples 3-4 are shown in Figures 1 and 2.

Even if VLDL and LDL were added to the dry analytical element of Example 2, the dry analytical element was not affected by the interference of such VLDL and LDL, and thus the measurement value of HDL-C did not become high. On the other hand, the HDL-C measurement of both Comparative examples 3 and 4 was affected by VLDL and LDL cholesterol, and as a result, the measurement value of HDL-C became high.

### Example 3: Dry analytical element for measurement ofHDL cholesterol

In the components of the dry analytical element of Example 1, only the concentration of the MES buffer differed from that of Example 1. The concentration of the MES buffer was 0.1 mmol/m², instead of 10 mmol/m².

### Example 4: Dry analytical element for measurement of HDL cholesterol

In the components of the dry analytical element of Example 1, only the concentration of the MES buffer differed from that of Example 1. The concentration of the MES buffer was 0.5 mmol/m², instead of 10 mmol/m².

### Example 5: Dry analytical element for measurement of HDL cholesterol

In the components of the dry analytical element of Example 1, only the concentration of the MES buffer differed from that of Example 1. The concentration of the MES buffer was 2.0 mmol/m², instead of 10 mmol/m².

### Example 6: Dry analytical element for measurement of HDL cholesterol

In the components of the dry analytical element of Example 1, only the concentration of the MES buffer differed from that of Example 1. The concentration of the MES buffer was 20.0 mmol/m², instead of 10 mmol/m².

### Example 7: Dry analytical element for measurement of HDL cholesterol

In the components of the dry analytical element of Example 1, only the concentration of the MES buffer differed from that of Example 1. The concentration of the MES buffer was 40.0 mmol/m², instead of 10 mmol/m².

### Example 8: Evaluation of HDL cholesterol measurement

HDL cholesterol was measured by the same method as the measurement method of Example 2, with the exception that the dry analytical element of Example 3 was used instead of that of Example 1.

### Example 9: Evaluation of HDL cholesterol measurement

HDL cholesterol was measured by the same method as the measurement method of Example 2, with the exception that the dry analytical element of Example 4 was used instead of that of Example 1.

### Example 10: Evaluation of HDL cholesterol measurement

HDL cholesterol was measured by the same method as the measurement method of Example 2, with the exception that the dry analytical element of Example 5 was used instead of that of Example 1.

### Example 11: Evaluation of HDL cholesterol measurement

HDL cholesterol was measured by the same method as the measurement method of Example 2, with the exception that the dry analytical element of Example 6 was used instead of that of Example 1.

### Example 12: Evaluation of HDL cholesterol measurement

HDL cholesterol was measured by the same method as the measurement method of Example 2, with the exception that the dry analytical element of Example 7 was used instead of that of Example 1.

### Comparative example 5: Dry analytical element for measurement of HDL cholesterol

In the components of the dry analytical element of Example 1, only the concentration of the MES buffer differed from that of Example 1. The concentration of the MES buffer was 60 mmol/m², instead of 10 mmol/m².

### Comparative example 6: Dry analytical element for measurement of HDL cholesterol

In the components of the dry analytical element of Example 1, only the concentration of the MES buffer differed from that of Example 1. The concentration of the MES buffer was 80 mmol/m², instead of 10 mmol/m².

### Comparative example 7: Evaluation of HDL cholesterol measurement

HDL cholesterol was measured by the same method as the measurement method of Example 2, with the exception that the dry analytical element of Comparative example 5 was used instead of that of Example 1.

### Comparative example 8: Evaluation of HDL cholesterol measurement

HDL cholesterol was measured by the same method as the measurement method of Example 2, with the exception that the dry analytical element of Comparative example 6 was used instead of that of Example 1.

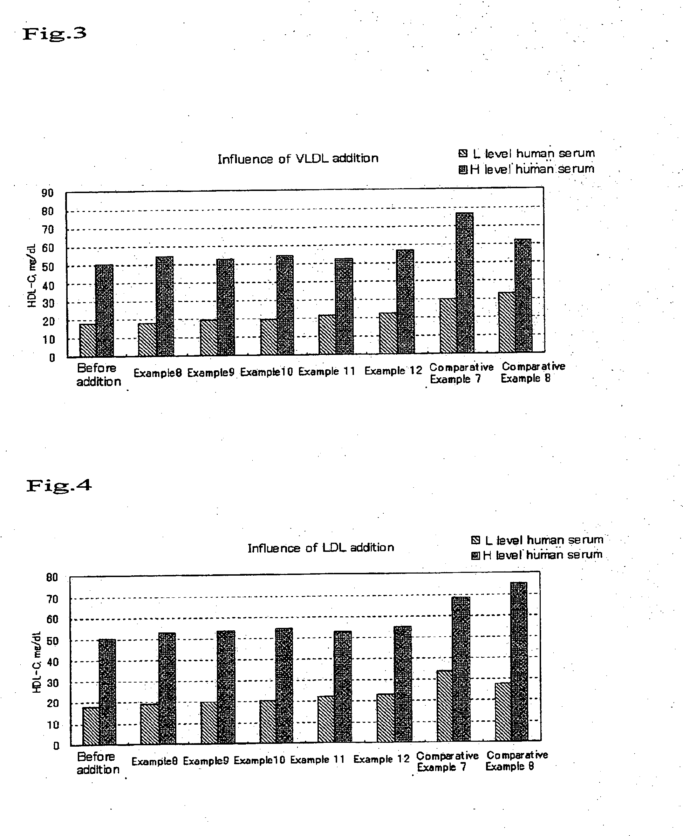

The HDL-C measurement and VLDL/LDL interference of each of Examples 8 to 12 and Comparative examples 7 and 8 are shown in Figures 3 and 4.

In the case of the dry analytical elements of Examples 8 to 12, even if VLDL and LDL were added thereto, the HDL-C value did not become high. As far as the concentration of the MES buffer was between 0.1 and 40 mmol/m², HDL-C was selectively measured. On the other hand, in the case of the concentration of the buffer of each of Comparative examples 7 and 8, the interference of VLDL and LDL increased, and thus the measurement of HDL-C could not be favorably carried out

### Example 13: Dry analytical element for measurement of HDL cholesterol

In the components of the dry analytical element of Example 1, only the concentration of the Emulgen A90 differed from that of Example 1. The concentration of the Emulgen A90 was 2.1 g/m², instead of 6.2 g/m².

### Example 14: Dry analytical-element for measurement ofHDL cholesterol

In the components of the dry analytical element of Example 1, only the concentration of the Emulgen A90 differed from that of Example 1. The concentration of the Emulgen A90 was 10.3 g/m², instead of 6.2 g/m².

### Example 15: Dry analytical element for measurement ofHDL cholesterol

In the components of the dry analytical element of Example 1, only the concentration of the Emulgen A90 differed from that of Example 1. The concentration of the Emulgen A90 was 14.5 g/m², instead of 6.2 g/m².

### Example 16: Dry analytical element for measurement ofHDL cholesterol

In the components of the dry analytical element of Example 1, only the concentration of the Emulgen A90 differed from that of Example 1. The concentration of the Emulgen A90 was 20.6 g/m², instead of 6.2 g/m².

### Example 17: Dry analytical element for measurement ofHDL cholesterol

A gelatin aqueous solution was coated to a 180-µm polyethylene terephthalate colorless transparent smooth film previously coated with gelatin, resulting in a thickness after drying of 14 µm, and the film was then dried. Subsequently, water was supplied in an amount of approximately 30 g/m² to the entire surface of the aforementioned film, so as to wet the film. Thereafter, a 36-gauge tricot knitting fabric made of polyester spun yarn corresponding to 50 deniers was slightly pressed and stacked thereon, followed by drying. Subsequently, an aqueous solution with a composition as described below was coated onto the fabric, followed by drying.

| | |
|---|---|
| MES buffer (pH 6.6) | 10 mmol/m² |
| Cholesterol esterase (derived from *Schizophyllum commune)* | 3.7 kU/m² |
| Cholesterol oxidase (derived from Pseudomonas sp.) | 1.1 kU/m² |
| Peroxidase | 60 kU/m² |
| 4-aminoantipyrine (manufactured by Wako Pure Chemical Industries, Ltd.) | 0.3 g/m² |
| DAOS (manufactured by DOJINDO Laboratories) | 0.5 g/m² |
| Emulgen B66 (manufactured by Kao Corp.) | 6.2 g/m² |
| Pluronic F-88 (manufactured by ADEKA Corp.) | 1.3 g/m² |
| Dextran sulfate (MW: 36,000 to 50,000) (manufactured by Wako Pure Chemical Industries, Ltd.) | 1.4 g/m² |
| Magnesium chloride hexahydrate (manufactured by Wako Pure Chemical Industries, Ltd.) | 8.8 g/m² |

### Example 18: Dry analytical element for measurement ofHDL cholesterol

In the components of the dry analytical element of Example 17, only the concentration of the Emulgen B66 differed from that of Example 17. The concentration of the Emulgen B66 was 10.3 g/m², instead of 6.2 g/m².

### Example 19: Dry analytical element for measurement ofHDL cholesterol

In the components of the dry analytical element of Example 17, only the concentration of the Emulgen B66 differed from that of Example 17. The concentration of the Emulgen B66 was 14.5 g/m², instead of 6.2 g/m².

### Example 20: Dry analytical element for measurement ofHDL cholesterol

In the components of the dry analytical element of Example 17, only the concentration of the Emulgen B66 differed from that of Example 17. The concentration of the Emulgen B66 was 20.6 g/m², instead of 6.2 g/m².

### Example 21: Evaluation ofHDL cholesterol measurement

HDL cholesterol was measured by the same method as the measurement method of Example 2, with the exception that the dry analytical element of Example 13 was used instead of that of Example 1.

### Example 22: Evaluation of HDL cholesterol measurement

HDL cholesterol was measured by the same method as the measurement method of Example 2, with the exception that the dry analytical element of Example 14 was used instead of that of Example 1.

### Example 23: Evaluation ofHDL cholesterol measurement

HDL cholesterol was measured by the same method as the measurement method of Example 2, with the exception that the dry analytical element of Example 15 was used instead of that of Example 1.

### Example 24: Evaluation of HDL cholesterol measurement

HDL cholesterol was measured by the same method as the measurement method of Example 2, with the exception that the dry analytical element of Example 16 was used instead of that of Example 1.

### Example 25: Evaluation of HDL cholesterol measurement

HDL cholesterol was measured by the same method as the measurement method of Example 2, with the exception that the dry analytical element of Example 17 was used instead of that of Example 1.

### Example 26: Evaluation of HDL cholesterol measurement

HDL cholesterol was measured by the same method as the measurement method of Example 2, with the exception that the dry analytical element of Example 18 was used instead of that of Example 1.

### Example 27: Evaluation of HDL cholesterol measurement

HDL cholesterol was measured by the same method as the measurement method of Example 2, with the exception that the dry analytical element of Example 19 was used instead of that of Example 1.

### Example 28: Evaluation of HDL cholesterol measurement

HDL cholesterol was measured by the same method as the measurement method of Example 2, with the exception that the dry analytical element of Example 20 was used instead of that of Example 1.

### Comparative example 9: Dry analytical element for measurement ofHDL cholesterol

In the components of the dry analytical element of Example 1, only the concentration of the Emulgen A90 differed from that of Example 1. The concentration of the Emulgen A90 was 1.0 g/m², instead of 6.2 g/m².

### Comparative example 10: Dry analytical element used in measurement ofHDL cholesterol

In the components of the dry analytical element of Example 17, only the concentration of the Emulgen B66 differed from that of Example 17. The concentration of the Emulgen B66 was 1.0 g/m², instead of 6.2 g/m².

### Comparative example 11: Evaluation ofHDL cholesterol measurement

HDL cholesterol was measured by the same method as the measurement method of Example 2, with the exception that the dry analytical element of Comparative example 9 was used instead of that of Example 1.

### Comparative example 12: Evaluation of HDL cholesterol measurement

HDL cholesterol was measured by the same method as the measurement method of Example 2, with the exception that the dry analytical element of Comparative example 10 was used instead of that of Example 1.

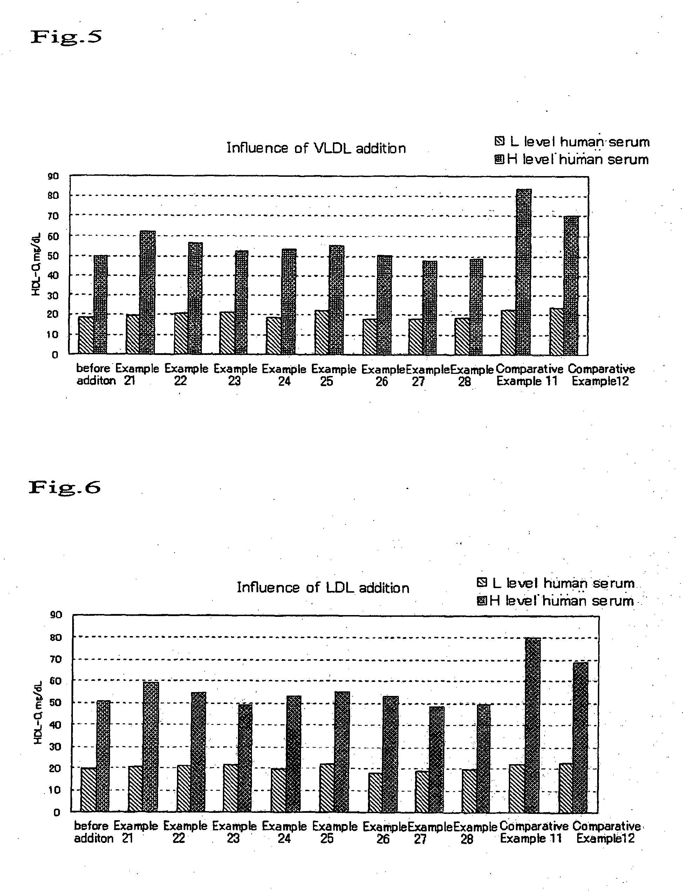

The HDL-C measurement and VLDL/LDL interference of each of Examples 21 to 28 and Comparative examples 11 and 12 are shown in Figures 5 and 6.

In the case of the dry analytical elements of Examples 21 to 28, even if VLDL and LDL were added thereto, the measurement was hardly affected thereby, and thus the HDL-C value did not become so high. As far as the amount of a nonionic surfactant selectively solubilizing HDL was within the range between 2.1 and 20.3 g/m², the interference of VLDL and LDL was low. If the amount of such a nonionic surfactant was 20.3 g/m² or greater, it became difficult to prepare it Thus, a concentration of greater than 20.3 g/m² was not evaluated. In the amount of the surfactant of each of Comparative examples 11 and 12, the inference of VLDL and LDL became high, and there was no selectivity for HDL-C.

## Claims

1. A dry analytical element for the measurement of a high density lipoprotein cholesterol, which has at least one layer comprising an nonionic surfactant of 3 to 22 g/m², a buffer of 0.1 to 40 mmol/m², dextran sulfate with a molecular weight of 36,000 to 50,000, a divalent metal ion and reagents for measuring high density lipoprotein cholesterol, on a support.

2. The dry analytical element for the measurement of a high density lipoprotein cholesterol according to claim 1, wherein the nonionic surfactant is a single type of nonionic surfactant, or a mixture of two or more types of nonionic surfactants.

3. The dry analytical element for the measurement of a high density lipoprotein cholesterol according to claim 1, wherein the nonionic surfactant is a polyoxyethylene derivative and/or a polyoxyethylene-polyoxypropylene condensate.

4. The dry analytical element for the measurement of a high density lipoprotein cholesterol according to claim 1, wherein one of the nonionic surfactants is a nonionic surfactant that selectively solubilizes a high density lipoprotein.

5. The dry analytical element for the measurement of a high density lipoprotein cholesterol according to claim 1, wherein one of the nonionic surfactants is a nonionic surfactant that inhibits dissolution of lipoproteins other than the high density lipoprotein.

6. The dry analytical element for the measurement of a high density lipoprotein cholesterol according to claim 1, wherein the buffer is present in at least one layer.

7. The dry analytical element for the measurement of a high density lipoprotein cholesterol according to claim 1, wherein the nonionic surfactant is present in at least one layer.

8. The dry analytical element for the measurement of a high density lipoprotein cholesterol according to claim 1, wherein the dextran sulfate is present in at least one layer.

9. The dry analytical element for the measurement of a high density lipoprotein cholesterol according to claim 1, which comprises at least cholesterol esterase and cholesterol oxidase as reagents for measuring the high density lipoprotein cholesterol.

10. The dry analytical element for the measurement of a high density lipoprotein cholesterol according to claim 1, which further comprises peroxidase and chromogens as reagents for measuring the high density lipoprotein cholesterol.

## Patentansprüche

1. Trockenes analytisches Element zur Messung von hochdichtem Lipoproteincholesterin, das mindestens eine Schicht auf einem Träger hat, die 3 bis 22 g/m² nichtionisches Tensid, 0,1 bis 40 mmol/m² Puffer, Dextransulfat mit einem Molekulargewicht von 36.000 bis 50.000, bivalentes Metallion und Reagenzien zur Messung von hochdichtem Lipoproteincholesterin umfasst.

2. Trockenes analytisches Element zur Messung von hochdichtem Lipoproteincholesterin nach Anspruch 1, in dem das nichtionische Tensid ein einziger Typ nichtionisches Tensid ist oder einer Gemisch aus zwei oder mehreren Typen von nichtionischen Tensiden ist.

3. Trockenes analytisches Element zur Messung von hochdichtem Lipoproteincholesterin nach Anspruch 1, in dem das nichtionische Tensid ein Polyoxyethylen-Derivat und/oder ein Polyoxyethylen-Polyoxypropylen-Kondensat ist.

4. Trockenes analytisches Element zur Messung von hochdichtem Lipoproteincholesterin nach Anspruch 1, in dem eines der nichtionischen Tenside ein nichtionisches Tensid ist, das selektiv ein hochdichtes Lipoprotein solubilisiert.

5. Trockenes analytisches Element zur Messung von hochdichtem Lipoproteincholesterin nach Anspruch 1, in dem eines der nichtionischen Tenside ein nichtionisches Tensid ist, das die Auflösung von anderen Lipoproteinen als das hochdichte Lipoprotein hemmt.

6. Trockenes analytisches Element zur Messung von hochdichtem Lipoproteincholesterin nach Anspruch 1, in dem der Puffer in mindestens einer Schicht vorhanden ist.

7. Trockenes analytisches Element zur Messung von hochdichtem Lipoproteincholesterin nach Anspruch 1, in dem das nichtionische Tensid in mindestens einer Schicht vorhanden ist.

8. Trockenes analytisches Element zur Messung von hochdichtem Lipoproteincholesterin nach Anspruch 1, in dem das Dextransulfat in mindestens einer Schicht vorhanden ist.

9. Trockenes analytisches Element zur Messung von hochdichtem Lipoproteincholesterin nach Anspruch 1, das mindestens Cholesterinesterase und Cholesterinoxidase als Reagenzien zur Messung von hochdichtem Lipoproteincholesterin enthält.

10. Trockenes analytisches Element zur Messung von hochdichtem Lipoproteincholesterin nach Anspruch 1, das weiterhin Peroxidase und Chromogene als Reagenzien zur Messung von hochdichtem Lipoproteincholesterin enthält.

## Revendications

1. Élément analytique sec pour la mesure du cholestérol lié aux lipoprotéines de haute densité, qui a au moins une couche comprenant un tensioactif non ionique de 3 à 22 g/m², un tampon de 0,1 à 40 mmol/m², du sulfate de dextrane avec un poids moléculaire allant de 36000 à 50000, un ion métallique divalent et des réactifs pour mesurer le cholestérol lié aux lipoprotéines de haute densité, sur un support.

2. Élément analytique sec pour la mesure du cholestérol lié aux lipoprotéines de haute densité selon la revendication 1, dans lequel le tensioactif non ionique est un seul type de tensioactif non ionique, ou un mélange de deux types ou plus de tensioactifs non ioniques.

3. Élément analytique sec pour la mesure du cholestérol lié aux lipoprotéines de haute densité selon la revendication 1, dans lequel le tensioactif non ionique est un dérivé de polyoxyéthylène et/ou un condensat de polyoxyéthylène-polyoxypropylène.

4. Élément analytique sec pour la mesure du cholestérol lié aux lipoprotéines de haute densité selon la revendication 1, dans lequel l'un des tensioactifs non ioniques est un tensioactif non ionique qui solubilise de manière sélective une lipoprotéine de haute densité.

5. Élément analytique sec pour la mesure du cholestérol lié aux lipoprotéines de haute densité selon la revendication 1, dans lequel l'un des tensioactifs non ioniques est un tensioactif non ionique qui inhibe la dissolution de lipoprotéines autres que la lipoprotéine de haute densité.

6. Élément analytique sec pour la mesure du cholestérol lié aux lipoprotéines de haute densité selon la revendication 1, dans lequel le tampon est présent dans au moins une couche.

7. Élément analytique sec pour la mesure du cholestérol lié aux lipoprotéines de haute densité selon la
revendication 1, dans lequel le tensioactif non ionique est présent dans au moins une couche.

8. Élément analytique sec pour la mesure du cholestérol lié aux lipoprotéines de haute densité selon la revendication 1, dans lequel le sulfate de dextrane est présent dans au moins une couche.

9. Élément analytique sec pour la mesure du cholestérol lié aux lipoprotéines de haute densité selon la revendication 1, qui comprend au moins une cholestérol estérase et une cholestérol oxydase en tant que réactifs pour mesurer le cholestérol lié aux lipoprotéines de haute densité.

10. Élément analytique sec pour la mesure du cholestérol lié aux lipoprotéines de haute densité selon la revendication 1, qui comprend en outre une peroxydase et des chromogènes en tant que réactifs pour mesurer le cholestérol lié aux lipoprotéines de haute densité.
